# EUROPEAN PATENT APPLICATION

(11) **EP 4 101 507 A1**
(43) Date of publication of application: **14.12.2022**
(21) Application number: 21765052.2
(22) Date of filing: 05.03.2021
(51) Int. Cl.: A61P 37/08, A61Q 11/00, A23K 10/12, A23K 10/16, A23K 10/18, A23L 33/135, A61K 8/9728, A61K 35/747

(54) **COMPOSITION CONTAINING LACTIC ACID BACTERIA**

(30) Priority: 06.03.2020 JP 2020038610
(71) Applicant: School Corporation, Azabu Veterinary Medicine Educational Institution, Sagamihara-shi, Kanagawa 252-5201 (JP); ITEA Inc., Bunkyo-ku, Tokyo 113-0001 (JP)
(72) Inventor: SAKAGUCHI Masahiro, Sagamihara-shi, Kanagawa 252-5201 (JP); UCHIYAMA Jumpei, Sagamihara-shi, Kanagawa 252-5201 (JP); FUKUYAMA Tomoki, Sagamihara-shi, Kanagawa 252-5201 (JP)
(74) Representative: Isarpatent
(86) International application number: PCT/JP2021/008596
(87) International publication number: WO 2021/177432

(57) **Abstract**

The present invention is intended to provide a lactic acid bacterium-containing composition that is effective for suppression of the onset of allergies. More specifically, the present invention relates to a composition comprising lactic acid bacteria belonging to *Lactobacillus animalis*, wherein the composition is provided in the form of, for example, a food or beverage composition, a pharmaceutical composition, a feed composition, a sanitary composition or the like. Furthermore, the present invention provides a method for preventing the onset of allergies.

## Description

### Technical Field

The present invention relates to a lactic acid bacterium-containing composition having an anti-allergic effect.

### Background Art

The onset of allergic disease is controlled by interaction of various factors, such as, for example, genetic factors and environmental factors (e.g. growing environment in infancy, intestinal bacterial flora, etc.).

Intestinal bacteria are considered to give influence on the development of the immune system of a host thereof (Non Patent Literature 1). Examples of intestinal bacteria that are somewhat associated with allergic disease may include lactic acid bacteria. The environment in which people live with their pet animals in their infancy is considered to be important as an environmental factor for allergic disease. For example, several reports have suggested that microbial crosstalk between humans and pet animals, which is generated by allowing people in their infancy to come into contact with pet animals (dogs, cats, etc.), should suppress the onset of allergies (Non Patent Literatures 2 and 3). Fujimura et al. have reported that the percentage of the lactic acid bacterium, *Lactobacillus johnsonii*, has significantly increased in the intestinal bacterial flora of mice exposed to house dust in the environment in which a dog has been kept, and thus that *Lactobacillus johnsonii* plays an important role for suppression of allergic airway disease (Non Patent Literature 4).

On the other hand, Taylor et al. have administered *Lactobacillus acidophilus* to babies born to mothers having allergic disease until the babies had 6 months old, and thus, they have examined the influence of the lactic acid bacterium on the onset of allergies. As a result, at the time point of 1 year after the birth, a significant difference was not found, in terms of the incidence rate of atopic dermatitis, between an administration group and a placebo administration group, and it was demonstrated that the IgE antibody positive frequency was rather higher in the *Lactobacillus acidophilus* administration group, than in the placebo administration group (Non Patent Literature 5).

As mentioned above, it has been suggested that lactic acid bacteria give some influence on allergic disease. However, conflicting results are given (i.e. Non Patent Literature 5 shows negative results, whereas Non Patent Literature 4 shows positive results), and thus, what bacterial species are more effective and practical for suppression of allergies needs to be further clarified.

### Citation List

### Non Patent Literature

Non Patent Literature 1: Macphers and Harris, Nat Rev Immunol. 4: 478-485, 2004.
Non Patent Literature 2: Hesselmar et al., Clin Exp Allergy. 29: 611-617, 1999.
Non Patent Literature 3: Fall et al., JAMA Pediatr. 169 e153219, 2015
Non Patent Literature 4: Fujimura et al., Proc Natl Aca Sci USA 14: 805-810, 2014.
Non Patent Literature 5: Tylor et al., J Allergy Clin Immuno. 119: 184-191, 2007.

### Summary of Invention

### Technical Problem

Under the above-described circumstances, it is an object of the present invention to provide a lactic acid bacterium-containing composition that is effective for suppression of the onset of allergies

### Solution to Problem

The present inventors have conceived that intestinal bacteria give some influence on the immune system of a host dog. Thus, the inventors have compared between healthy dogs and atopic dermatitis dogs, in terms of intestinal bacterial flora, and have then selected bacteria that were predominant in the healthy dogs. Specifically, among 240 strains separated from the feces of the healthy dogs, 184 strains corresponded to the above-selected bacteria, and these bacteria were subjected to phylogenetic classification analysis with 16sRNA gene. As a result, 4 genera (i.e. genus Bacteroide, genus Enterococcus, genus Lactobacillus, and genus Streptococcus), 13 species were identified. Then, from these identified species, genus Lactobacillus, *Lactobacillus animalis* (*L. animalis*), which had not been reported regarding relationship with the onset of allergies so far, was used as an analysis target.

The present inventors have orally administered *L. animalis* to atopic dermatitis mouse models and allergic asthma mouse models. As a result, it was found that *L*. *animalis* exhibited the effect of suppressing various symptoms caused by allergies. Moreover, it was also found that *L. animalis* exhibited a higher allergy-inhibitory effect than *Lactobacillus johnsonii* (*L. johnsonii*), which had been previously reported to have an allergy-inhibitory effect.

The present invention has been completed based on the aforementioned findings.

Specifically, the present invention includes the following (1) to (7).
(1) A composition, comprising lactic acid bacteria belonging to *Lactobacillus animalis* or processed bacterial products thereof.
(2) The composition according to the above (1), wherein the lactic acid bacteria are *Lactobacillus animalis* strains deposited with Accession No. NITE BP-03137, Accession No. NITE BP-03138, Accession No. NITE BP-03139, and/or Accession No. NITE BP-03140.
(3) The lactic acid bacterium or composition according to the above (1) or (2), which has anti-allergic activity.
(4) The composition according to any one of the above (1) to (3), wherein the composition is a food or beverage composition.
(5) The composition according to any one of the above (1) to (3), wherein the composition is a pharmaceutical composition.
(6) The composition according to any one of the above (1) to (3), wherein the composition is a feed composition.
(7) The composition according to any one of the above (1) to (3), wherein the composition is a sanitary composition.

### Advantageous Effects of Invention

According to the present invention, provided is a lactic acid bacterial species that exhibits a higher allergy-suppressing effect than conventionally reported lactic acid bacterial species.

### Brief Description of Drawings

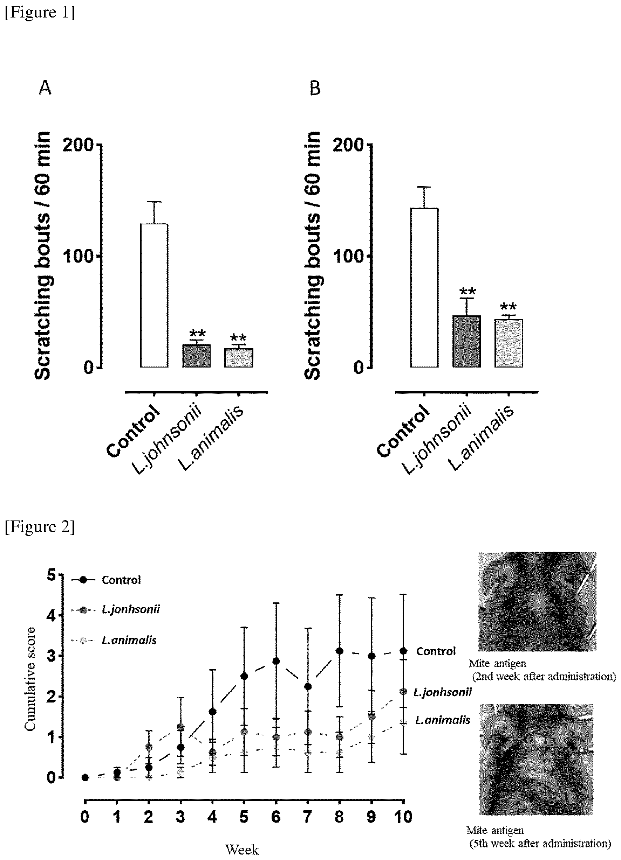

[Figure 1] Figure 1 shows analysis results (1) obtained using atopic dermatitis models. The number of scratching behaviors per hour in mouse models (n = 8) was measured, 4 weeks (A) and 7 weeks (B) after initiation of the transdermal administration of a mite allergen. The data show the mean value, and the error bar shows a standard error. ^{∗∗}P < 0.01.
[Figure 2] Figure 2 shows analysis results (2) obtained using atopic dermatitis models. The figure shows the results obtained by scoring the skin symptoms of mouse models (n = 8) every week, until 10 weeks after initiation of the transdermal administration of a mite allergen (left graph). The data show the mean value, and the error bar shows a standard error. In addition, photographs of the skin symptoms of the mouse models after administration of the mite allergen are shown (right view).

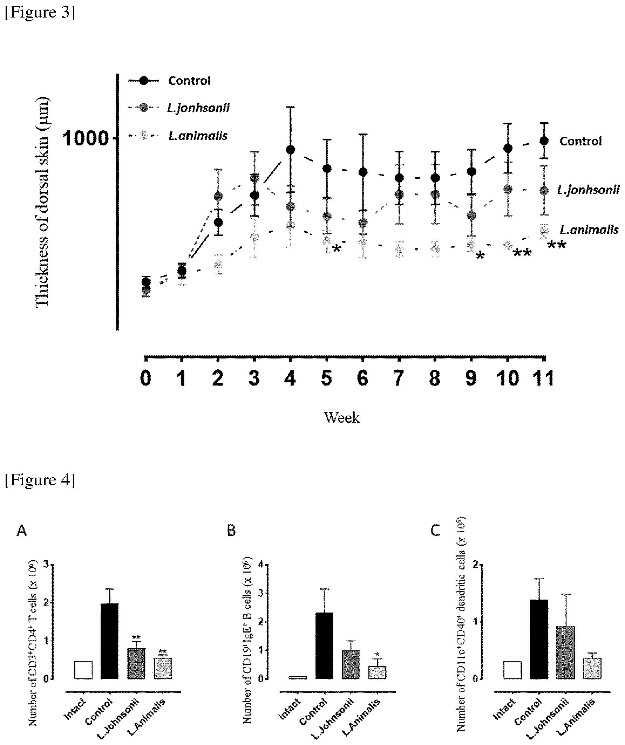

[Figure 3] Figure 3 shows analysis results (3) obtained using atopic dermatitis models. The figure shows the results obtained by measuring the thickness of the dorsal skin of each mouse model (n = 8) every week, until 10 weeks after initiation of the transdermal administration of a mite allergen. The data show the mean value, and the error bar shows a standard error. ^{∗∗}P < 0.01, ^{∗}P < 0.05.
[Figure 4] Figure 4 shows analysis results (4) obtained using atopic dermatitis models. The figure shows the results obtained by counting the numbers of CD3⁺CD4⁺ T cells (A), CD19⁺IgE⁺ B cells (B), and CD11c⁺CD40⁺ dendritic cells (C), which were present in the auricular lymph nodes collected from the mouse models (n = 8), on the following day of the final administration of a mite allergen. The data show the mean value, and the error bar shows a standard error. ^{∗∗}P < 0.01, ^{∗}P < 0.05. "Intact" indicates the results obtained using mice not-treated with the mite, and "Control" indicates the results obtained using mouse models not-administered with lactic acid bacteria (the same applies in the following figures).

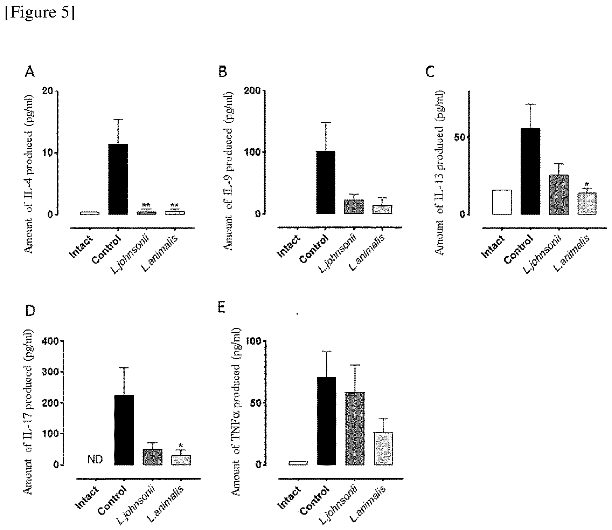

[Figure 5] Figure 5 shows analysis results (5) obtained using atopic dermatitis models. The amounts of various types of cytokines produced in the auricular lymph nodes collected from the mouse models (n = 8), on the following day of the final administration of a mite allergen (A: IL-4, B: IL-9, C: IL-13, D: IL-17, and E: TNFα). "ND" indicates not detected (below the detection limit). The data show the mean value, and the error bar shows a standard error. ^{∗∗}P < 0.01, ^{∗}P < 0.05.

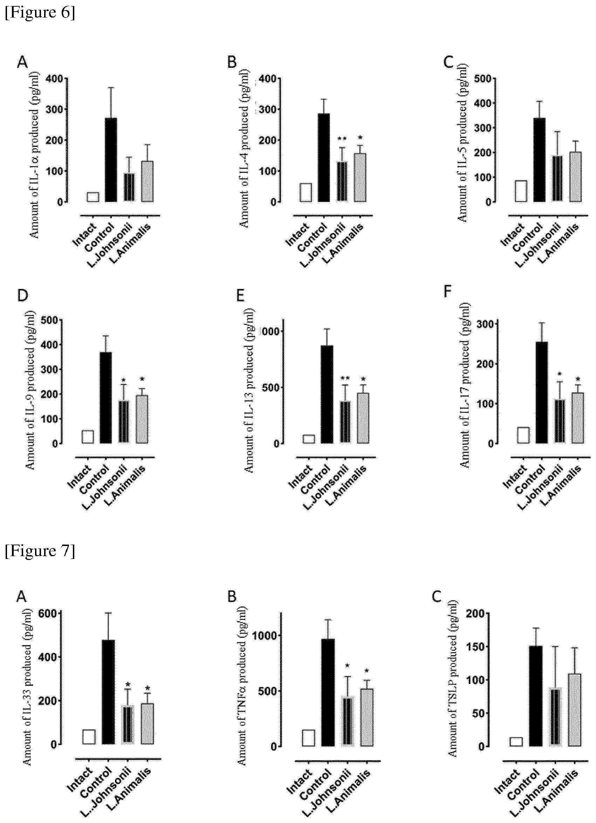

[Figure 6] Figure 6 shows analysis results (6) obtained using atopic dermatitis models. On the following day of the final administration of a mite allergen, the amounts of various types of cytokines produced in the auricular skin tissues collected from the mouse models (n = 8) were measured (A: IL-1α, B: IL-4, C: IL-5, D: IL-9, E: IL-13, and F: IL-17). The data show the mean value, and the error bar shows a standard error. ^{∗∗}P < 0.01, ^{∗}P < 0.05.
[Figure 7] Figure 7 shows analysis results (7) obtained using atopic dermatitis models. On the following day of the final administration of a mite allergen, the amounts of various types of cytokines produced in the auricular skin tissues collected from the mouse models (n = 8) were measured (A: IL-33, B: TNFα, and C: TSLP). The data show the mean value, and the error bar shows a standard error. ^{∗}P < 0.05.

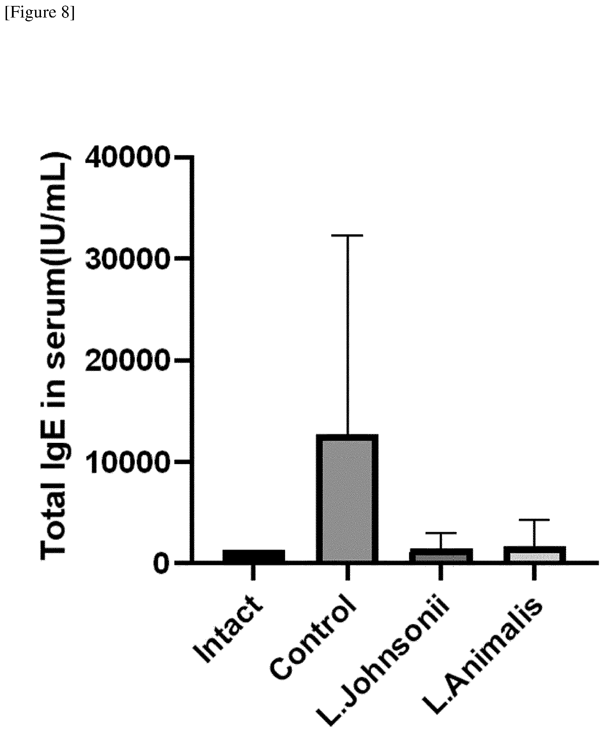

[Figure 8] Figure 8 shows analysis results (8) obtained using atopic dermatitis models. On the following day of the final administration of a mite allergen, serum was prepared from the blood collected from each mouse model (n = 8), and the total IgE amount contained in the serum was then measured. The error bar shows a standard error.

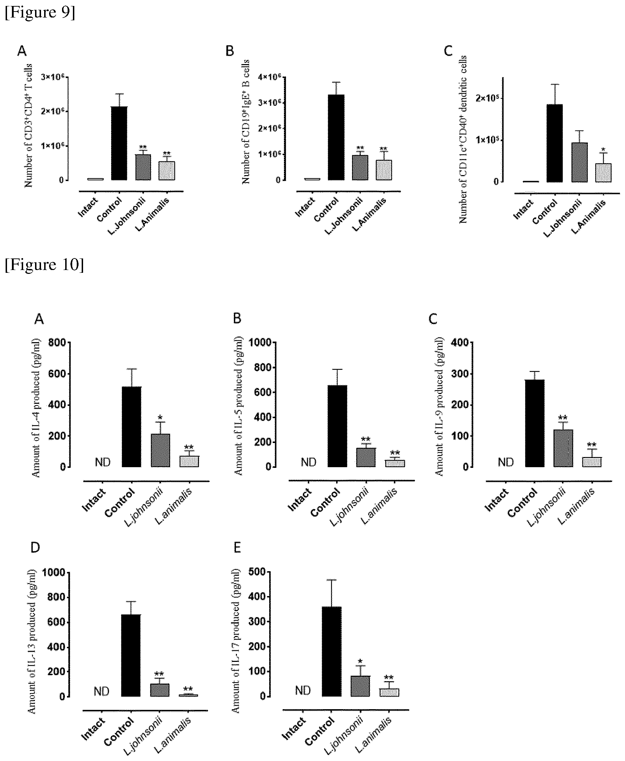

[Figure 9] Figure 9 shows analysis results (1) obtained using allergic asthma models. The figure shows the results obtained by counting the numbers of CD3⁺CD4⁺ T cells (A), CD19⁺IgE⁺ B cells (B), and CD11c⁺CD40⁺ dendritic cells (C), which were present in the hilar lymph nodes collected from the mouse models (n = 8), on the following day of the final induction with a mite allergen. The data show the mean value, and the error bar shows a standard error. ^{∗∗}P < 0.01, ^{∗}P < 0.05.
[Figure 10] Figure 10 shows analysis results (2) obtained using allergic asthma models. The amounts of various types of cytokines produced in the hilar lymph nodes collected from the mouse models (n = 8) were measured on the following day of the final induction with a mite allergen (A: IL-4, B: IL-5, C: IL-9, D: IL-13, and E: IL-17). "ND" indicates not detected (below the detection limit). The data show the mean value, and the error bar shows a standard error. ^{∗∗}P < 0.01, ^{∗}P < 0.05.

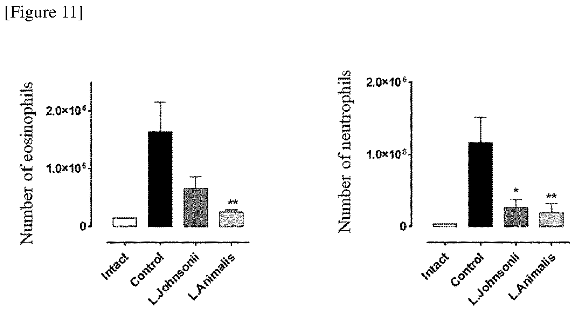

[Figure 11] Figure 11 shows analysis results (3) obtained using allergic asthma models. The figure shows the results obtained by counting the numbers of eosinophils (A) and neutrophils (B) contained in the bronchoalveolar lavage fluid collected from the mouse models (n = 8) on the following day of the final induction with a mite allergen. The data show the mean value, and the error bar shows a standard error. ^{∗∗}P < 0.01, ^{∗}P < 0.05.

### Description of Embodiments

A first embodiment of the present invention relates to a composition, comprising lactic acid bacteria belonging to *Lactobacillus animalis* or processed bacterial products thereof (hereinafter also referred to as "the composition of the present invention"). Preferred examples of the *Lactobacillus animalis* used in the embodiment of the present invention may include: a strain whose gene sequence has been registered with NCBI Accession No. NZ_AYYW00000000.1; and strains, which were deposited under the National Institute of Technology and Evaluation (NITE), Biotechnology Center, Patent Microorganisms Depositary (NPMD) (2-5-8, Kazusa Kamatari , Kisarazu-shi, Chiba-ken, Japan; postal code: 292-0818) on February 21, 2020 (original deposit date), with Accession No. NITE P-03137 (ID: L11-2), Accession No. NITE P-03138 (ID: L13-1), Accession No. NITE P-03139 (ID: L41-1), and Accession No. NITE P-03140 (ID: M08-1). Besides, the strains specified with Accession No. NITE P-03137, Accession No. NITE P-03138, Accession No. NITE P-03139, and Accession No. NITE P-03140 were then transferred from the original deposition to an international deposition under the provisions of the Budapest Treaty (the date of issue of "Deposit Receipt of the Original Deposit" and "Life Certificate": March 4, 2021). The accession numbers of the transferred strains are Accession No. NITE BP-03137, Accession No. NITE BP-03138, Accession No. NITE BP-03139, and Accession No. NITE BP-03140, respectively. The aforementioned deposited strains are available from the aforementioned preservation institution.

The method of culturing *Lactobacillus animalis* is not particularly limited, and any culture method may be applied, as long as it is a method of culturing lactic acid bacteria that is generally selected by those skilled in the art. For example, *Lactobacillus animalis* can be cultured at a culture temperature of 20°C to 50°C, and preferably 25°C to 40°C, under anaerobic conditions.

The medium used to proliferate *Lactobacillus animalis* is not particularly limited, and a medium generally selected by those skilled in the art can be used. Such a medium may be, for example, a medium comprising carbon sources (glucose, galactose, mannose, lactose, sucrose, cellobiose, trehalose, etc.), nitrogen sources (ammonia, ammonium sulfate, ammonium chloride, ammonium nitrate, etc.), inorganic salts (sodium chloride, potassium chloride, potassium sulfate, magnesium sulfate, calcium chloride, calcium nitrate, etc.), and organic components (peptone, yeast extract, meat extract, soybean powder, etc.), at a composition suitable for the stain species. An MRS medium or an LBS medium can be preferably used.

Examples of the processed bacterial product of the present invention may include a cell culture product and a cell fermented product. The state of the lactic acid bacteria contained in the processed bacterial product may be either the state of dead cells or the state of living cells. Examples of the processed bacterial product may further include, but are not limited to: lactic acid bacteria that have been subjected to heating, pasting, drying, freezing, lysis, crushing, extraction, etc.; and supernatants, etc. obtained by removing solids from cell crushed products, cell culture products, and fermented products.

Moreover, the composition of the present invention may comprise other subjects, as well as *Lactobacillus animalis* or a processed bacterial product thereof. Such other substances are not particularly limited, and the composition of the present invention may comprise, for example, lactose, glucose, mannitol, sucrose, dextrin, cyclodextrin, starch, cellulose, collagen, citric acid, acetic acid, common salts, vitamins, and the like.

The composition of the present invention has the effect of suppressing the onset of allergies, and the composition of the present invention can be provided in the form of, for example, a food or beverage composition, a pharmaceutical composition, a feed composition, a sanitary composition or the like, depending on the intended purpose thereof. However, the composition of the present invention is not limited to the aforementioned compositions.

When the composition of the present invention is a pharmaceutical composition, the dosage form thereof is not particularly limited. Examples of the dosage form of the pharmaceutical composition may include a tablet, a capsule, a granule, a powder agent, a syrup, a liquid preparation, a suppository, and an injection. These preparations are produced according to ordinary methods. Besides, the liquid preparation may be a preparation that is dissolved or suspended in water or another suitable solvent, when it is used. In addition, the tablet and the granule may be coated according to publicly known methods. The injection is prepared by dissolving the antibody of the present invention or a functional fragment thereof in water. The antibody of the present invention or a functional fragment thereof may also be dissolved in a normal saline or a glucose solution, as necessary, and a buffer agent or a preservative may also be added to the injection.

When the composition of the present invention is provided as a food or beverage composition, the form thereof is not particularly limited. Examples of the form of the food or beverage composition may include drinks such as soft drinks or nutritional drinks, confectioneries such as candies, gum, jelly, cream or ice cream, dairy products such as milk drink, fermented milk, drink yogurt or butter, and other supplements.

When the composition of the present invention is provided as a sanitary composition, the form thereof is not particularly limited. Examples of the form of the sanitary composition may include toothpaste, skin cream, soap, shampoo, a cosmetic product, aerosol, mist, and a coating agent. It may also be a sanitary composition for use in non-human animals.

The composition of the present invention may also be a feed composition that can be administered to non-human animals, or a pharmaceutical composition for use in animals. The animal feed applied herein may include supplements for animals that are given as favorite items to animals, as well as feeds that are administered as sources of nutrients necessary for animals.

A second embodiment of the present invention relates to a method for preventing the onset of allergies, comprising administering the pharmaceutical composition of the present invention to a subject (hereinafter also referred to as "the preventive method of the present invention").

Herein, the term "prevention" means a treatment for the purpose of inhibiting, in advance, the onset of allergies.

The subject of the preventive method of the present invention is not particularly limited, as long as it is any given animal classified into mammals. Examples of the subject may include humans, pet animals such as dogs, cats or rabbits, and livestock animals such as bovines, pigs, sheep or horses. The "mammals" are particularly preferably humans and dogs.

When an English translation of the present description includes singular terms with the articles "a," "an," and "the," these terms include not only single items but also multiple items, unless otherwise clearly specified from the context.

Hereinafter, the present invention will be further described in the following examples. However, these examples are only illustrative examples of the embodiments of the present invention, and thus, are not intended to limit the scope of the present invention.

### Examples

### 1. Experimental methods

### 1-1. Separation of lactic acid bacteria from feces and phylogenetic classification

From 240 bacterial strains separated from the feces of healthy dogs (16 dogs), 184 strains that were predominant in the intestinal bacterial flora of the healthy dogs over the intestinal bacterial flora of atopic dermatitis dogs were selected. The selected 184 strains were subjected to phylogenetic classification analysis. Specifically, using an MRS medium or an LBS medium, the feces collected from each dog was subjected to a static culture under anaerobic conditions at 37°C overnight. After completion of the culture, bacteria were separated using an MRS or LBS plate medium. Purification was carried out, at least, three times. Phylogenetic classification of the bacteria was carried out based on the 16S rRNA sequence. As a result, 4 genera (i.e. genus Bacteroide, genus Enterococcus, genus Lactobacillus, and genus Streptococcus), 13 species were identified. Then, from these identified species, genus Lactobacillus, *Lactobacillus animalis* (*L. animalis*), which had not been reported so far, regarding relationship with the onset of allergies, was used as an analysis target. Besides, as a positive control used in the present Examples, *Lactobacillus johnsonii* (*L. johnsonii*), which had been suggested to be associated with the onset of allergies, was used. The *L. animalis* used in the present Examples was obtained by mixing the strains deposited with Accession No. NITE BP-03137, Accession No. NITE BP-03138, Accession No. NITE BP-03139, and Accession No. NITE BP-03140 at a mixing ratio of 1 : 1 : 1 : 1 (the number of cells).

### 1-2. Analysis using disease mouse models

All animal experiments used in the present Examples were carried out under the approval of the Animal Experiment Expert Committee, Azabu University of Tokyo.

### 1-2-1. Atopic dermatitis models

An experiment was carried out using 6-week-old female NC/Nga mice (Charles River Laboratories Japan, Inc.). The mice were acclimated for 1 week, and thereafter, the mice were orally administered with *L. animalis* or *L. johnsonii* in an amount of 0.2 ml (10⁹) per mouse for 2 weeks. Thereafter, sensitization with a mite allergen (*Dermatophagoides farinae*) was started. Before the sensitization with the mite allergen, the skin of the dorsocervical portion was dehaired, and tape stripping was then carried out on the dorsocervical portion 10 times. After that, 30 µl (0.25 mg/ml) of a mite suspension was administered to both pinnae of the mice via transdermal administration by pipetting 10 µl each of the mite suspension. The mite allergen was administered to the mice twice a week for 12 weeks, and the monitoring of itching behavior, the scoring of skin symptoms, and the measurement of the thickness of the dorsal skin were carried out every week.

With regard to the monitoring of itching behavior, among the behaviors of the mice recorded by a video camera, the behavior of the mice to bite or scratch the mite allergen-coated site (i.e. pinna and dorsal skin) with a hind limb, a forelimb or tongue was defined to be one itching behavior, and the number of itching behaviors for 60 minutes was recorded.

With regard to the skin findings, crust/ulcer formation and redness were scored for each of the pinna portion and the dorsal skin (the degree of symptoms was scored using 0 to 4 scales), and the cumulative value was then evaluated as a cumulative score.

The thickness of the dorsal skin was measured using a vernier caliper once a week.

On the following day of the final administration of a mite allergen, blood was collected from the mice under inhalation anesthesia of Isoflurane, and the mice were then euthanized. Thereafter, an auricular lymph node and the skin of pinna were collected.

Serum was separated from the blood, and the total IgE amount in the blood was then quantified according to an ELISA method.

Regarding the auricular lymph node, after separation of single cells, cells were separated and obtained based on cell surface antigens according to flow cytometry. Among the obtained cells, helper T cells (CD3⁺CD4⁺ T cells) were cultured in the presence of an anti-CD3 antibody and an anti-CD28 antibody (VERITAS Corporation) for 24 to 96 hours, and thereafter, the amounts of various types of cytokines in the culture supernatant were measured according to an ELISA method.

The collected skin of pinna was frozen with liquid nitrogen, and was then homogenized in 500 µl of PBS, using an electric homogenizer, and the amounts of various types of cytokines in the obtained supernatant were then measured according to an ELISA method.

### 1-2-2. Allergic asthma models

An experiment was carried out using 6-week-old female BALB/c mice (Charles River Laboratories Japan, Inc.). The mice were acclimated for 1 week, and thereafter, the mice were orally administered with *L. animalis* or *L. johnsonii* in an amount of 0.2 ml (10⁹) per mouse for 2 weeks. Thereafter, sensitization with a mite allergen (*Dermatophagoides farinae*) was started. Sensitization with the mite allergen was carried out by pipetting 30 µl (1 mg/ml) of a mite suspension to the mice under inhalation anesthesia of Isoflurane via nasal drop administration. Administration of a mite allergen was continuously carried out once a week for 3 weeks. On the 4th week, by the same method as described above, 5 µl (0.2 mg/ml) of the mite suspension was administered to the mice via nasal drop induction under inhalation anesthesia of Isoflurane for 3 continuous days. On the following day of the final induction with the mite allergen, blood was collected from the mice under inhalation anesthesia of Isoflurane, and the mice were then euthanized. Thereafter, a hilar lymph node and a bronchoalveolar lavage fluid were collected from the mice.

The bronchoalveolar lavage fluid was used to count the numbers of eosinophils and neutrophils contained in the lavage fluid.

Regarding the hilar lymph node, after separation of single cells, cells were separated and obtained based on cell surface antigens according to flow cytometry. Among the obtained cells, helper T cells (CD3⁺CD4⁺ T cells) were cultured in the presence of the anti-CD3 antibody and the anti-CD28 antibody for 24 to 96 hours, and thereafter, the amounts of various types of cytokines in the culture supernatant were measured according to an ELISA method.

### 1-3. Statistical analysis

From the obtained data, the mean value and the standard error were calculated in each group, and a multiple comparison test was carried out on all groups for every experiment. In the multiple comparison test, a test for variance was carried out according to a Bartlett method or the like, and in the case of variance, a Dunnett's test was applied. Regarding individual test items, the presence or absence of a statistically significant difference among individual groups was analyzed at levels of significance of 5% and 1%.

### 2. Results

### 2-1. Atopic dermatitis models

Atopic dermatitis is a pathological condition, in which itching and skin inflammation are alternatively expressed and the symptoms are then deteriorated. Hence, the itching behaviors of the mouse models per hour were measured once a week, immediately after administration of a mite allergen, and the influence of lactic acid bacteria on the itching was examined.

From the analysis results of the mouse models, to which the mite allergen had been administered to develop atopic dermatitis, it was found that the number of scratching behaviors was significantly reduced in *L. animalis-*administered mice and *L*. *johnsonii-*administered mice, compared with control mice (which had not been administered with lactic acid bacteria) (Figures 1A and 1B).

Figure 2 shows the results obtained by scoring atopic skin symptoms during the mite allergen administration period. The expression of atopic symptoms widely varied depending on individual mice, and a statistically significant difference was not observed. However, the degree of reduction in skin symptoms was higher in the *L*. *animalis* administration group and the *L. johnsonii* administration group, than in the control group. In addition, when the *L. animalis* administration group was compared with the *L. johnsonii* administration group, the degree of reduction in the symptoms was higher in the *L. animalis* administration group than in the *L. johnsonii* administration group.

In the atopic dermatitis, together with deterioration of the symptoms, epidermal thickening and/or cellular infiltration progress, and the thickness of the skin increases. Thus, the thickness of the dorsal skin of the mouse models was measured. As a result, the thickness of the skin was more significantly reduced in the *L. animalis* administration group and the *L. johnsonii* administration group, than in the control group. Among others, a reduction in the thickness of the skin in the *L. animalis* administration group was statistically significant, and a clear reduction was observed in the *L. animalis* administration group, compared with the *L. johnsonii* administration group (Figure 3).

After completion of the final dissection, the auricular lymph node was collected, and the numbers of immune cells (helper T cells (CD3⁺CD4⁺ T cells), IgE-positive B cells (CD19⁺IgE⁺ B cells) and activated dendritic cells (CD11c⁺CD40⁺ dendritic cells)), which were associated with allergies and were present in the lymph node, were counted according to flow cytometry. As a result, a reduction in the numbers of all of the aforementioned cells was observed in the *L. animalis* administration group and the *L. johnsonii* administration group. In particular, the numbers of the helper T cells and the IgE-positive B cells were significantly reduced in the *L. animalis* administration group and the *L. johnsonii* administration group, compared with in the control group, and among others, a significant reduction in the numbers of the cells was observed in the *L. animalis* administration group (Figure 4).

Subsequently, helper T cells collected from the auricular lymph node were cultured in the presence of CD3 and CD28 antibodies, and the amounts of inflammatory cytokines (IL-4, IL-9, IL-13, IL-17, and TNFα) produced in the cells were then measured. As a result, the produced amounts of all of the inflammatory cytokines were significantly reduced in the *L. animalis* administration group and the *L*. *johnsonii* administration group, compared with in the control group (Figure 5). In particular, a more significant reduction in the produced amounts of the inflammatory cytokines was observed in the *L. animalis* administration group, compared with in the *L. johnsonii* administration group.

The amounts of inflammatory cytokines (IL-1α, IL-4, IL-5, IL-9, IL-13, and IL-17) in the auricular skin tissues were measured. As a result, as with the results of the auricular lymph node, a significant reduction in the amounts of the cytokines was observed in the *L. animalis* administration group and the *L. johnsonii* administration group (Figure 6). Moreover, the results obtained by quantifying the amounts of cytokines associated with itching (IL-33, TNFα, and TSLP (thymic stromal lymphopoietin)) in the auricular skin tissues are shown in Figure 7. Regarding TSLP, a significant reduction in the amount of this cytokine was not observed. On the other hand, it was observed that the produced amounts of keratinocyte-derived cytokines such as IL-33 and TNFα were significantly reduced in the *L*. *animalis* administration group and the *L. johnsonii* administration group, compared with in the control group.

Subsequently, the total IgE amount in the serum prepared from the mouse models was measured. As a result, it was observed that the total IgE amount in the serum was significantly reduced in the *L. animalis* administration group and the *L*. *johnsonii* administration group (Figure 8).

### 2-2. Allergic asthma models

After completion of the final dissection of allergic asthma mouse models, the hilar lymph node was collected, and then, as in the case of the atopic dermatitis models, the numbers of allergy-related immune cells (helper T cells, IgE-positive B cells, and activated dendritic cells) were analyzed according to flow cytometry. As a result, with regard to all of the allergy-related immune cells, it was observed that the numbers of individual cells in the hilar lymph node were significantly reduced in the *L*. *animalis* administration group and the *L. johnsonii* administration group. In particular, a significant reduction in the numbers of the cells was found in the *L*. *animalis* administration group (Figure 9).

Subsequently, the helper T cells collected from the hilar lymph node were cultured in the presence of CD3 and CD28 antibodies, and the amounts of inflammatory cytokines (IL-4, IL-5, IL-9, IL-13, and IL-17) produced in the cells were then measured. As a result, with regard to all of the inflammatory cytokines, the produced amounts of the inflammatory cytokines were significantly reduced in the *L*. *animalis* administration group and the *L. johnsonii* administration group, compared with in the control group (Figure 10). In particular, the produced amounts of the inflammatory cytokines were significantly reduced in the *L. animalis* administration group, rather than in the *L. johnsonii* administration group.

Upon the final dissection of the allergic asthma mouse models, the lung was washed using PBS, and the numbers of eosinophils and neutrophils in the obtained lavage fluid (bronchoalveolar lavage fluid, BALF) were then counted according to flow cytometry (Figure 11). With regard to all of the cell species, the numbers of the cells were reduced in the *L. animalis* administration group and the *L. johnsonii* administration group, and in particular, a significant reduction in the numbers of the cells was observed in the *L*. *animalis* administration group.

### Industrial Applicability

The present invention provides a composition exhibiting the effect of suppressing the onset of allergies, and it is expected that the present composition will be utilized in the field of manufacturing foods and beverages, as well as the medical field and the veterinary field.

### Accession Numbers

Accession No. NITE BP-03137
Accession No. NITE BP-03138
Accession No. NITE BP-03139
Accession No. NITE BP-03140

## Claims

1. A composition, comprising lactic acid bacteria belonging to *Lactobacillus animalis* or processed bacterial products thereof.

2. The composition according to claim 1, wherein the lactic acid bacteria are *Lactobacillus animalis* strains deposited with Accession No. NITE BP-03137, Accession No. NITE BP-03138, Accession No. NITE BP-03139, and/or Accession No. NITE BP-03140.

3. The lactic acid bacterium or composition according to claim 1 or 2, which has anti-allergic activity.

4. The composition according to any one of claims 1 to 3, wherein the composition is a food or beverage composition.

5. The composition according to any one of claims 1 to 3, wherein the composition is a pharmaceutical composition.

6. The composition according to any one of claims 1 to 3, wherein the composition is a feed composition.

7. The composition according to any one of claims 1 to 3, wherein the composition is a sanitary composition.
